# EUROPEAN PATENT SPECIFICATION

(11) **EP 2 004 072 B1**
(45) Date of publication and mention of the grant of the patent: **11.09.2013**
(21) Application number: 07775213.7
(22) Date of filing: 11.04.2007
(51) Int. Cl.: A61B 17/34, A61B 17/3205, A61B 17/00, A61M 25/00, A61B 17/32, A61B 18/14

(54) **APPARATUS FOR ENDOSCOPIC RESECTION OF TISSUE**
VORRICHTUNG ZUR ENDOSKOPISCHEN GEWEBERESEKTION
APPAREIL DE RÉSECTION ENDOSCOPIQUE DE TISSUS

(30) Priority: 13.04.2006 US 791668 P
(43) Date of publication of application: 24.12.2008
(73) Proprietor: Cook Medical Technologies LLC, Bloomington, IN 47404 (US)
(72) Inventor: SKERVEN, Gregory J., Kernersville, NC 27284 (US); KARPIEL, John A., Winston-Salem, NC 27106 (US)
(74) Representative: Garratt, Peter Douglas
(86) International application number: PCT/US2007/008970
(87) International publication number: WO 2007/120727

(56) References cited:
- WO-A-02/35986
- US-A- 5 183 463
- US-A1- 2001 034 527
- US-A1- 2005 056 292
- US-A1- 2005 149 099
- US-A1- 2005 165 437

## Description

### TECHNICAL FIELD

The present invention relates generally to enhanced apparatus for performing an endoscopic mucosal resection or submucosal dissection of tissue.

### BACKGROUND INFORMATION

Diagnostic and therapeutic gastrointestinal endoscopy are commonly used to gain access to the digestive tract for the purpose of removing tissue. One technique for obtaining tissue for biopsies is an endoscopic mucosectomy procedure, also known as endoscopic mucosal resection ("EMR"). The EMR procedure may be a useful tool for providing a tissue specimen for surgical pathology.

The EMR procedure also may be used for curative purposes to remove sessile benign tumors and intramucosal cancers, and in particular, EMR is a well-accepted treatment for early gastric cancer without lymph node metastasis. During curative removal of a mucosal lesion, it is desirable to perform "en-bloc resection" of the lesion, i.e., removal in one piece. If the lesion is removed in a piecemeal fashion, it is believed that rates of local tumor recurrence may be increased. Further, assessment of fragmented tissue may be more difficult than assessment of unfragmented tissue.

During an EMR procedure, it may be desirable to mark and subsequently resect a portion of tissue surrounding a lesion to ensure that the lesion is completely resected in an en-bloc fashion. In addition to removing the mucosal tissue, a portion of the submucosa also may be removed.

A typical EMR procedure involves identifying the mucosal lesion using an endoscope. The boundaries of the lesion may be marked to facilitate removal. A fluid, such as saline or sodium hyaluronate, may be injected into the submucosal layer just beneath the lesion to help the lesion protrude away from the remaining healthy tissue. A snare may be used to resect the mucosal tissue that includes the lesion. A forceps or snare may be used to grasp and remove the resected tissue via the endoscope.

One reported drawback associated with conventional EMR procedures is that the snaring method tends to yield piecemeal resection of a lesion, which may ruin the histopathologic assessment of the lesion. Further, EMR procedures generally are not recommended for large lesions, e.g., over 2 cm in diameter.

Recently, a technique called endoscopic submucosal dissection ("ESD") has been developed in which mucosal lesions are removed by the dissection of submucosa under the lesion using an incision device, such as an endoscopic knife. Reference is directed to US 2005/149099. (This is the prior art on which the characterising form of the appended claims is based). The ESD procedure may facilitate resection of larger lesions and yield improved en-bloc resection, as compared to a conventional EMR procedure.

In view of the drawbacks of current technology, it is desirable to develop apparatus and methods for an EMR or ESD procedure that may efficiently remove mucosal and/or submucosal tissue in unfragmented portions in a relatively short period of time without inducing significant patient trauma.

### SUMMARY

The present invention is set forth in the appended claims and provides apparatus for performing EMR and ESD procedures. In a first embodiment, the apparatus comprises a catheter having proximal and distal ends and a balloon disposed near the distal end of the catheter. During an ESD procedure, a portion of the distal end of the catheter is configured to be inserted beneath a section of mucosal tissue having a lesion. The balloon is configured to be inflated to lift the mucosal tissue in an upward direction, thereby facilitating removal of the tissue comprising the lesion.

In a method of operation, the catheter is delivered to a target site through a working channel of an endoscope. A needle knife may be used to make markings in the tissue to define the boundaries of the lesion prior to incision of the tissue. In a next step, a needle may pierce the mucosal tissue to deliver fluid, such as saline, to the submucosal layer beneath the target tissue site. This fluid injection causes the mucosal tissue having the lesion to bulge outward, i.e., away from the muscularis propria. In a next step, the needle knife may be used to incise the tissue to be removed, e.g., by applying electrical current to the distal tip of the needle knife.

In a next step, the balloon on the distal end of the catheter is positioned at least partially beneath the mucosal tissue to be removed. The balloon is then inflated, which may facilitate removal or detachment of the incised tissue. If desired, a surgeon may advance the needle knife through the catheter to further incise submucosal tissue while the balloon is in the inflated state.

The needle is a separate component that is configured to be advanced through a lumen of the catheter. The catheter may comprise a standard distal tip, and the needle may be selectively advanced independent of the catheter to pierce tissue.

In further alternative embodiments, a flushing fluid may be provided to a target site for various purposes, e.g., to flush away debris, cool off the needle knife, and so forth. The flushing fluid may be delivered through any number of pathways, such as a separate catheter lumen, or the working channel or an auxiliary lumen of an endoscope used in conjunction with the ESD procedure.

Other systems, features and advantages of the invention will be, or will become, apparent to one with skill in the art upon examination of the following figures and detailed description. It is intended that all such additional systems, methods, features and advantages be within the scope of the invention, and be encompassed by the following claims.

### BRIEF DESCRIPTION OF THE DRAWINGS

The invention can be better understood with reference to the following drawings and description. The components in the figures are not necessarily to scale, emphasis instead being placed upon illustrating the principles of the invention. Moreover, in the figures, like referenced numerals designate corresponding parts throughout the different views.

FIGS. 1A-1B are, respectively, a side view of a catheter provided in accordance with a first example, and a side-sectional view of the distal end of the catheter of FIG. IA.

FIG. 2 is a perspective view of the distal end of an endoscope that may be used in conjunction with the present invention.

FIGS. 3A-3B are, respectively, a side view and a top view of a method step that may be used.

FIGS. 4A-4B are, respectively, a side view and a top view of another method step that may be used.

FIG. 5 is a side view of a method step that may be used.

FIG. 6 is a side view of a method step that may be used.

FIG. 7 is a side view of a method step that may be used.

FIG. S is a side view of a method step that may be used.

FIG. 9 is a side view of the distal end of a catheter provided in accordance with an embodiment of the invention.

FIG. 10 is a cross-sectional view of the distal end of a catheter taken along line A-A of FIG. 9.

FIG. 11 is an alternative cross-sectional view of the distal end of a catheter taken along line A-A of FIG. 9.

FIG. 12 is a perspective view of the distal end of an endoscope and other components that may be used in conjunction with an alternative embodiment.

FIG. 13 is a perspective view of the distal end of an endoscope and other components that may be used in conjunction with a further alternative example.

### DETAILED DESCRIPTION OF THE PREFERRED EMBODIMENTS

In the present application, the term "proximal" refers to a direction that is generally towards a physician during a medical procedure, while the term "distal" refers to a direction that is generally towards a target site within a patent's anatomy during a medical procedure.

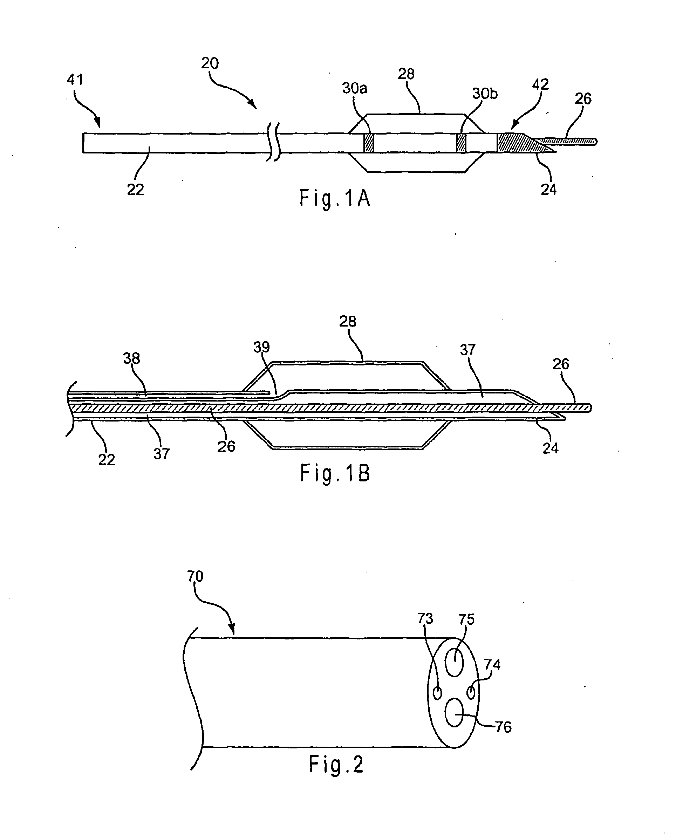

Referring now to FIGS. 1A-1B, a first example of a balloon catheter is described. In FIG. 1A, apparatus 20 comprises catheter 22 having proximal end 41 and distal end 42. Catheter 22 preferably comprises a flexible, tubular member that may be formed from one or more semi-rigid polymers. For example, catheter 22 may be manufactured from polyurethane, polyethylene, tetrafluoroethylene, polytetrafluoroethylene, perfluoalkoxl, fluorinated ethylene propylene, or the like. The catheter may have a length and an outer diameter sufficient to extend through working channel 76 of conventional endoscope 70 (see FIG. 2). For example, catheter 22 may comprise an outer diameter of about 6 to 7 French in order to fit within the working channel.

Catheter 22 also may comprise a hydrophilic coating (not shown) overlying its outer surface. The hydrophilic coating, when applied to the outer surface of catheter 22, imparts suppleness and kink resistance to the catheter. The hydrophilic coating also may provide a lubricated surface to facilitate movement through working channel 76 of endoscope 70.

Referring still to FIG. 1A, apparatus 20 further comprises needle 24, needle knife 26, and balloon 28. Balloon 28 is disposed on the distal end of catheter 22, as shown in FIGS. 1A-1B. Balloon 28 may comprise any balloon material and configuration that is known in the art, e.g., for performing balloon angioplasty, delivering a stent, or other interventional purpose. Balloon 28 may comprise a relatively compliant balloon material, for example, made of silicone or latex, or may comprise a relatively non-compliant balloon material, for example, made of nylon. The dimensions of balloon 28 may include a length of about 10mm and a diameter of about 10mm in an inflated state. Such dimensions are for reference purposes only and are not intended to be limiting, and the drawings in the figures are not necessarily given to scale.

Catheter 22 comprises first lumen 38, which is an inflation lumen. First lumen 38 spans from proximal end 41 of catheter 22 to aperture 39, which is disposed within the interior volume of balloon 28, as shown in FIG. 1B. Therefore, the provision of fluid such as air or saline via first lumen 38 may selectively inflate balloon 28. If desired, catheter 22 may also comprise one or more marker elements 30a and 30b, which may be used, for example, to help identify the position of balloon 28 for purposes described below.

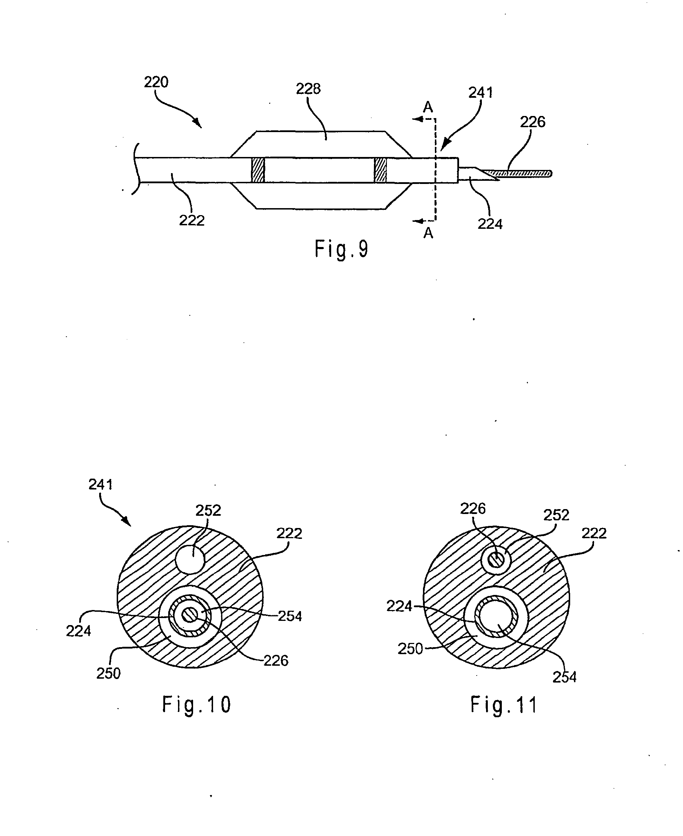

Apparatus 20 further comprises needle 24. As shown in the embodiment of FIGS. 1A-1B, needle 24 may be coupled to distal end 42 of catheter 22 to form a sharp, distal region configured to pierce through a portion of a patient's tissue. In FIGS. 1A-1B, needle 24 may be formed as an integral component with catheter 22, i.e., such that distal movement of catheter 22 causes distal advancement of needle 24. In this example, a relatively sharp needle, tip may be affixed to the distal tip of catheter 22, e.g., using an adhesive, to form a needle-shaped element at the distal end of the catheter. Alternatively, a separate needle configured to be inserted through a lumen of catheter 22 may be employed, such as needle 224 of FIG. 9 (discussed below).

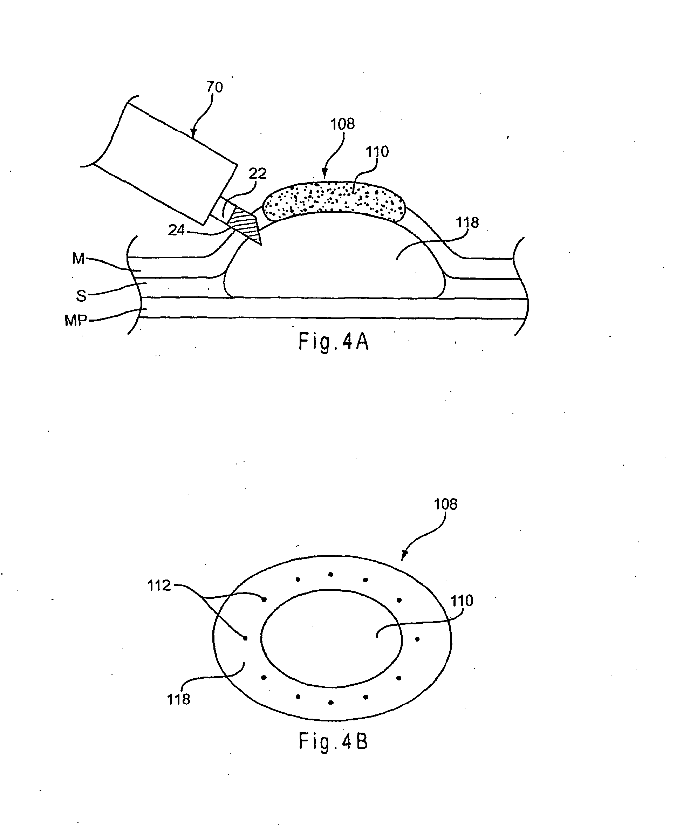

Regardless of whether needle 24 is coupled to the distal end of catheter 22, or configured to be disposed through a lumen of catheter 22, the needle is used to inject a biocompatible fluid, such as saline solution. As explained below with respect to FIG. 4A, this procedure causes the target tissue, e.g., a mucosal lesion, to elevate from the muscularis propria. Elevation of the target tissue facilitates removal of the lesion during an endoscopic mucosal resection procedure. The ability to remove the abnormal tissue without cutting into it enables a more accurate assessment of the tissue than would otherwise be possible if sampling a fragmented tissue sample. Furthermore, fragmented resection of early cancers may lead to a higher rate of local tumor recurrence.

Referring still to FIGS. 1A-1B, needle knife 26 may comprise any cutting instrument known in the art for performing tissue resection. Various needle knife configurations are known for performing mucosal resection and submucosal dissection of tissue. One exemplary needle knife is described in U.S. Patent Application Serial No 60/788,207, filed March 31, 2006.

In the embodiment of FIGS. 1A-1B, needle knife 26 is disposed through second lumen 37 of catheter 22. Needle knife 26 has an outer diameter that is smaller than an inner diameter of second lumen 37 to permit longitudinal movement of the needle knife within the lumen, and also permit injection of fluid through second lumen 37, i.e., around the needle knife, for purposes described below.

Referring now to FIG. 2, an exemplary endoscope, which may be used in conjunction with apparatus 20 during an EMR or ESD procedure, is described. Endoscope 70 may comprise any conventional endoscope having proximal and distal ends. In FIG. 2, only the distal end of endoscope 70 is shown. Endoscope 70 may comprise optical elements 73 and 74, which employ fiber optic components for illuminating and capturing an image distal to the endoscope. Further, endoscope 70 preferably comprises auxiliary lumen 75 and working channel 76. As noted above, working channel 76 preferably is sized to accommodate catheter 22 therein for purposes of longitudinally advancing the catheter within the working channel. It will be apparent to one skilled in the art that while one auxiliary lumen 75 and one working channel 76 are shown, endoscope 70 may comprises any number of lumens/channels.

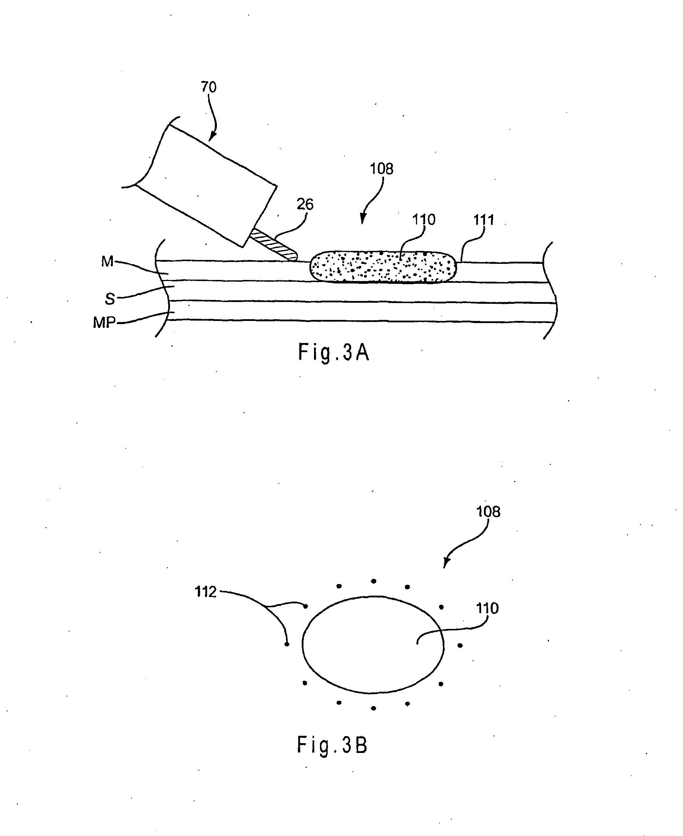

Referring now to FIGS. 3-8, a method for performing an EMR or ESD procedure is described. In a first step, endoscope 70 is maneuvered towards a target tissue site 108 using endoscopy techniques that are known in the art. For example, the device may be maneuvered into a patient's mouth, down through the esophagus and duodenum, and towards the target tissue site 108. Target tissue site 108 may comprise lesion 110, e.g., indicative of gastric cancer, which is fully or partially confined within mucosal tissue layer M. Beneath musoca. M, submusoca S and muscularis propria MP are present, as shown in FIG. 3A. It should be noted that during advancement of endoscope 70 to target tissue site 108, catheter 22 may be retracted within working channel 76 of endoscope 70 (see FIG. 2). Alternatively, catheter 22 may not yet be disposed within the endoscope during its advancement to the target site.

Once endoscope 70 is positioned adjacent target tissue site 108, a physician will examine whether incision markings are needed to define the boundaries of target tissue site 108. If the margins 111 of target tissue site 108 are not readily discernible, needle knife 26 may be loaded through second lumen 37 of catheter 22, which itself may be disposed within working channel 76 of endoscope 70. Needle knife 26 may then be advanced distal to endoscope 70 so as to engage the target tissue and create markings 112 around margins 111 of target tissue site 108, as depicted in FIG. 3B. High frequency current may be applied to the needle knife tip to create the markings. Such methods for creating markings are well known to those of ordinary skill in the art. Alternatively, markings 112 may be omitted where target tissue site 108 can readily be distinguished from tissue not intended to be cut.

In an alternative embodiment, needle knife 26 need not be advanced through second lumen 37 of catheter 22. For example, needle knife 26 instead may be advanced through auxiliary lumen 75 ofendoscope 70 (see FIG. 2).

Referring now to FIGS. 4A-4B, in a next step, the targeted mucosal tissue may be lifted with respect to muscularis propria MP to facilitate removal of lesion 110. Protrusion of target tissue site 108 may be achieved by injecting a fluid, such as physiological saline solution or sodium hyaluronate, through needle 24 and into the submucosa S. As explained above, needle 24 may be disposed at the distal end of catheter 22 and integrally coupled thereto. The injected fluid may flow through second lumen 37 of catheter 22. When needle knife 26 is disposed within catheter 22, the fluid may flow around the needle knife, prior to exiting through the distal tip of needle 24. Alternatively, as noted above, needle knife 26 may be disposed within auxiliary lumen 75, in which case injected fluid flows substantially unobstructed through second lumen 37 of catheter 22.

As shown in FIG. 4A, the fluid injection into submucosa S lifts target tissue site 108 from the underlying muscularis propria MP, thereby forming fluid pocket 118 in submucosal layer S. Fluid pocket 118 is shown from an elevated view in FIG. 4B. By elevating target tissue site 108 having lesion 110, a subsequent excision of lesion 110 is facilitated, as explained in greater detail below.

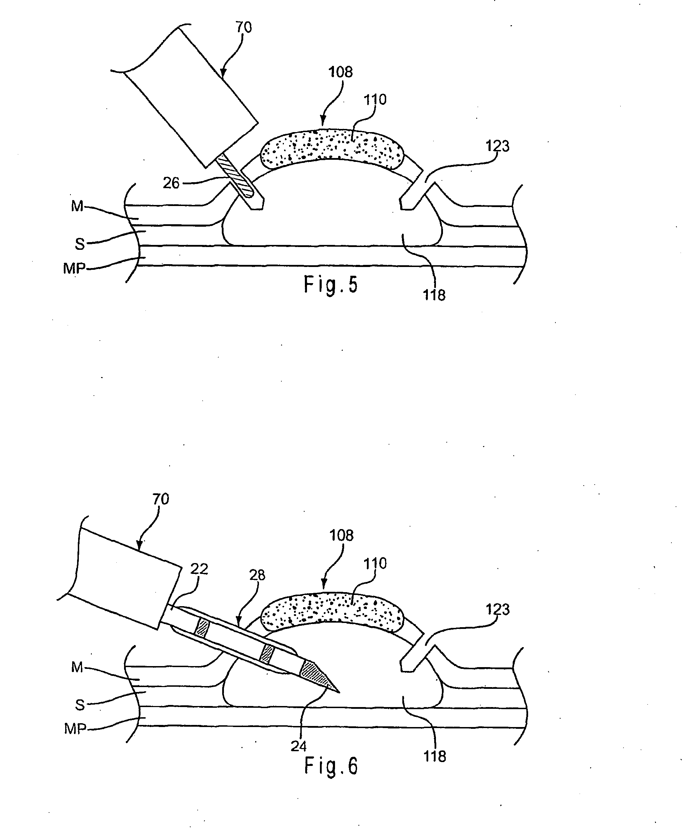

Referring now to FIG. 5, after target tissue site 108 has been sufficiently elevated, the process of creating a mucosal incision may begin. Needle 24 may be retracted proximally to be confined within working channel 76 of endoscope 70, and needle knife 26 may be advanced distally through second lumen 37 of catheter 22, as depicted in FIG. 5.

The mucosal incision may be made circumferentially around lesion 110 using needle knife 26, as depicted in FIG. 5. An electrosurgical generator (not shown) may be coupled to needle knife 26 to provide an electrical energy sufficient to incise the tissue. The incision preferably is performed at a predetermined distance into submucosa S, and at a predetermined angle with respect to muscularis propria MP.

Needle knife 26 may be fabricated from any electrically conductive material, including stainless steel. Alternatively, it may be fabricated from a shape memory alloy such as nitinol, as described in the above-referenced U.S. patent application Serial No. 60/788,207, filed March 31, 2006. Optionally, needle knife 26 may comprise a non-conductive portion at its tip, such as a hollow or ceramic region, which helps prevent the needle knife from cutting too far into tissue. Other safety mechanisms will be apparent to one skilled in the art.

Referring now to FIG. 6, after target tissue 108 has been incised, needle knife 26 is retracted to withdraw the distal end of the needle knife completely into second lumen 37 of catheter 22. Alternatively, if needle knife 26 is disposed through auxiliary lumen 75, it is retracted within that lumen.

In a next step, catheter 22 is advanced in a distal direction, beyond the distal end of endoscope 70, as shown in FIG. 6. Needle 24 pierces through mucosa M and into fluid pocket 118 within submucosa S. It should be noted that balloon 28 is in a deflated state to facilitate advancement of catheter 22 through working channel 76. At this time, catheter 22 preferably is positioned such that a portion of balloon 28 is disposed beneath a portion of target tissue site 108, as shown in FIG. 6.

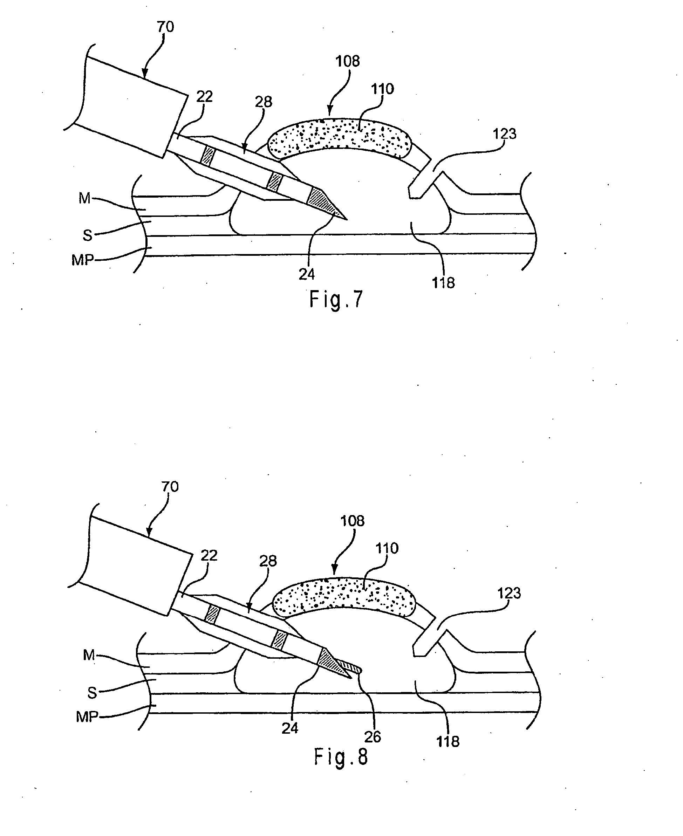

Upon proper positioning, balloon 28 is inflated by injecting an inflation fluid into first lumen 38, through aperture 39, and into the inner confines of balloon 28. In the expanded state, shown in FIG. 7, balloon 28 lifts up target tissue site 108 from beneath it, thereby facilitating resection of lesion 110. In particular, the inflated balloon may help dislodge the mucosal portion of target tissue site 108 away from submucosa S. In the process, portions of submucosa S also may be drawn away from muscularis propria MP. While the inflated balloon is depicted as being disposed under a relatively small portion of lesion 110 in FIGS. 7-8, it will be apparent that the balloon may be advanced further beneath the lesion prior to inflation.

Referring now to FIG. 8, while balloon 28 is inflated, needle knife 26 optionally may be advanced beyond the distal tip of needle 24, thereby dissecting submucosal tissue from within fluid pocket 118. Therefore, in addition to the mucosal resection procedure performed in FIG. 5, a submucosal dissection may be achieved in FIG. 8 to facilitate "en-bloc" removal of target tissue site 108.

Once the incised target tissue is sufficiently separated from its surrounding tissue, balloon 28 may be deflated and catheter 22 and needle knife 26 may be withdrawn. A retrieval device, such as a snare or forceps (not shown), then may be advanced through auxiliary lumen 75 or working lumen 76 to subsequently remove incised target tissue 108, which includes lesion 110. The endoscope then may be removed from the patient to complete the procedure.

Advantageously, by employing a balloon catheter during the ESD procedure, a surgeon may selectively inflate the balloon from beneath target tissue site 108 to help dislodge the incised mucosal tissue. Further, if the surgeon dissects submucosal tissue S using a needle knife, inflating balloon 28 may help hold surrounding tissue in place. Finally, as noted above, the submucosal dissection techniques described herein may promote "en-bloc" removal of lesion 110 to improve subsequent pathological assessment of the lesion.

Referring now to FIGS. 9-13, various alternative embodiments are shown. In FIG. 9, apparatus 220 according to the invention is shown comprising a catheter 222 having proximal and distal ends and balloon 228 disposed on the distal end. Apparatus 220 is similar to apparatus 20 of FIGS. 1A-1B, with a main exception that needle 224 is a separate component that is slidable witch respect to catheter 222. Needle 224 may be sized to longitudinally move within a lumen of catheter 222, e.g., lumen 250 of FIG. 10. Further, needle knife 226 may be configured to be disposed within inner lumen 254 of needle 224, as depicted in FIG. 10. Fluid that is injected via needle 224, such as saline, may flow around needle knife 226 in reaching a target site.

Alternatively, catheter 222 may comprise separate lumens for various purposes. For example, a first lumen may be used to inflate balloon 228, in a manner similar to lumen 38 of FIG. 1B. Further, a second lumen, such as lumen 250 of FIGS. 10-11, may be used to permit advancement of needle 224. Finally, a third lumen, such as lumen 252 of FIG. 11, may be used to permit advancement of needle knife 226; not according to the invention.

In the embodiment of FIG. 9, the preferred method steps for using apparatus 220 are similar to the methods steps for using apparatus 20, as described in FIGS. 3-8. However, it should be noted that the distal tip of catheter 222 is substantially flat. Therefore, during the fluid injection step shown in FIG. 4A, the distal tip of catheter 222 preferably is housed within working channel 76 of endoscope 70, while needle 224 is advanced beyond the distal ends of catheter 222 and endoscope 70 to engage and inject fluid into submucosa S. Further, during the balloon positioning and inflation steps described in FIGS. 6-7 above, needle 224 may be retracted within the confines of catheter 222 to enable a flat distal region during placement of the balloon catheter.

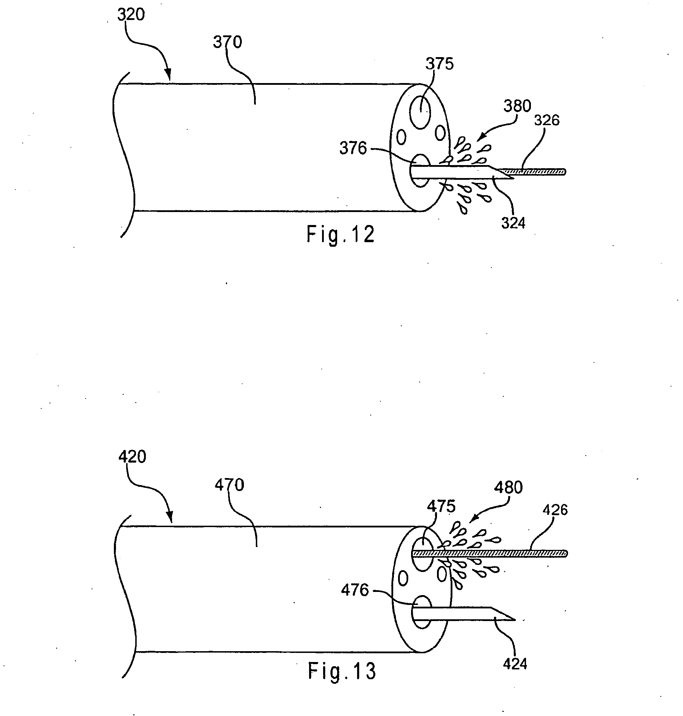

Referring now to FIGS. 12-13, further alternative embodiments of the present invention are described. In these embodiments, apparatus are provided for performing a flushable EMR or ESD procedure. In FIG. 12, apparatus 320 comprises needle 324 having needle knife 326 disposed therein. Needle 324 is disposed within working channel 376 of endoscope 370. Needle 324, needle knife 326 and endoscope 370 may be provided substantially in accordance with the embodiments described above.

In FIG. 12, fluid 380 may be delivered to a target site via working channel 376, i.e., the fluid may be delivered through the working channel in an annular space around the exterior of needle 324. Alternatively, fluid may be delivered through auxiliary lumen 375 of endoscope 370, either alone or in conjunction with delivery of fluid through working channel 376. In either case, the fluid that is delivered may be used to flush away debris, provide a cooling effect during operation of needle knife 326, and so forth.

Referring to FIG. 13, a variation of the embodiment of FIG. 12 is shown, wherein apparatus 420 comprises needle 424, which may be advanced through working channel 476, and needle knife 426, which may be advanced through auxiliary lumen 475 of endoscope 470. Flushing fluid may be delivered through auxiliary lumen 475, as shown in FIG. 13, or exclusively through working channel 476, or may be delivered through both auxiliary lumen 475 and working channel 476. It will be apparent that flushing fluid also may be provided through various other openings or ports disposed on the endoscope.

It will be appreciated that the embodiments described in FIGS. 1-11 above also may employ flushing fluid. For example, referring to FIG. 10, lumen 250 of catheter 222 may permit delivery of needle 224, while lumen 252 may be used exclusively for delivering a flushing fluid. Alternatively, needle knife 226 may be delivered through lumen 252, as depicted in FIG. 11, and flushing fluid may be delivered through lumen 252, i.e., around the needle knife. In yet a further alternative embodiment of FIG. 11, flushing fluid may be delivered via lumen 250, i.e., around the exterior surface of needle 224. Other variations will be apparent to those skilled in the art.

It will be appreciated that the apparatus and methods described hereinabove may be used to treat various types of lesions, e.g., large superficial tumors and intraepithelial neoplasms, in virtually any body cavity, such as the stomach, esophagus and colon.

While various embodiments of the invention have been described, it will be apparent to those of ordinary skill in the art that many more embodiments and implementations are possible within the scope of the invention. Accordingly, the invention is not to be restricted except in light of the attached claims.

## Claims

1. Apparatus suitable for performing mucosal resection of tissue, the apparatus comprising:
a catheter (222) having proximal and distal ends;
a balloon (228) disposed near the distal end of the catheter;
a needle (224) having a sharpened distal region configured to pierce through mucosal tissue and further dimensioned for injecting fluid to submucosal tissue;
a needle knife (226) operable to incise a section of mucosal tissue;
wherein a portion of the distal end of the catheter is configured to be inserted beneath the incised section of mucosal tissue and the balloon is configured to be inflated to lift a targeted mucosal layer of the tissue in an upward direction to facilitate removal of the tissue, **characterised in that** the needle is slidable within a first lumen (250) of the catheter; and **in that** the needle knife (226) is slidable within a lumen (254) of the needle (224).

2. The apparatus of claim 1 wherein the catheter has an outer diameter that is configured to be disposed through a working channel (376) of an endoscope.

3. The apparatus of claim 1 wherein the catheter comprises a second lumen (38) disposed between the proximal and distal ends of the catheter and configured to provide an inflation fluid to inflate the balloon.

4. The apparatus of claim 2 wherein flushing fluid is configured to be delivered through the working channel (376) of the endoscope.

## Patentansprüche

1. Vorrichtung, die zur Durchführung einer Schleimhautresektion von Gewebe geeignet ist und folgendes umfasst:
einen Katheter (222) mit proximalen und distalen Enden;
einen Ballon (228), der in der Nähe des distalen Endes des Katheters angeordnet ist;
eine Nadel (224), die eine angespitzte distale Region zum Durchstechen von Schleimhautgewebe aufweist und zur Injektion von Flüssigkeit in submukosales Gewebe bemessen ist;
ein Nadelmesser (226) zum Setzen einer Inzision in Schleimhautgewebe;
worin ein Teil des distalen Endes des Katheters zur Einführung unter der Schleimhautgewebeinzision konfiguriert ist und der Ballon aufgeblasen werden kann, um eine angezielte Schleimhautschicht des Gewebes in einer Aufwärtsrichtung zur Erleichterung der Entfernung von Gewebe anzuheben, **dadurch gekennzeichnet, dass** die Nadel in einem ersten Lumen (250) des Katheters gleiten kann; und dass das Nadelmesser (226) in einem Lumen (254) der Nadel (224) gleiten kann.

2. Vorrichtung nach Anspruch 1, worin der Katheter einen Außendurchmesser aufweist, der zur Durchführung durch einen Arbeitskanal (376) eines Endoskops konfiguriert ist.

3. Vorrichtung nach Anspruch 1, worin der Katheter ein zweites Lumen (38) umfasst, das zwischen den proximalen und distalen Enden des Katheters angeordnet ist und zur Zuführung einer Aufblasflüssigkeit zum Aufblasen des Ballons konfiguriert ist.

4. Vorrichtung nach Anspruch 2, worin Spülflüssigkeit zur Abgabe durch den Arbeitskanal (376) des Endoskops konfiguriert ist.

## Revendications

1. Appareil approprié pour réaliser une resection mucosique de tissus, l'appareil comprenant :
un cathéter (222) ayant des extrémités proximale et distale ;
un ballonnet (228) disposé à proximité de l'extrémité distale du cathéter ;
une aiguille (224) ayant une région distale aiguisée configurée de manière à percer à travers les tissus mucosiques et dimensionnée en outre pour injecter du fluide vers les tissus sous-mucosiques ;
un couteau d'aiguille (226) servant à inciser une section de tissus mucosiques ;
une partie de l'extrémité distale du cathéter étant configurée pour être insérée sous la section incisée de tissus mucosiques et le ballonnet étant configuré pour être gonflé de manière à soulever une couche mucosique cible des tissus dans une direction vers le haut afin de faciliter l'enlèvement des tissus, **caractérisé en ce que** l'aiguille peut coulisser à l'intérieur d'une première lumière (250) du cathéter ; et **en ce que** le couteau d'aiguille (226) peut coulisser à l'intérieur d'une lumière (254) de l'aiguille (224).

2. Appareil selon la revendication 1, dans lequel le cathéter a un diamètre extérieur qui est configuré de manière à être disposé à travers un canal de travail (376) d'un endoscope.

3. Appareil selon la revendication 1, dans lequel le cathéter comprend une deuxième lumière (38) disposée entre les extrémités proximale et distale du cathéter et configurée de manière à fournir un fluide de gonflage pour gonfler le ballonnet.

4. Appareil selon la revendication 2, dans lequel le fluide de rinçage est configuré de manière à être délivré à travers le canal de travail (376) de l'endoscope.
